# EUROPEAN PATENT APPLICATION

(11) **EP 3 988 652 A1**
(43) Date of publication of application: **27.04.2022**
(21) Application number: 20207983.6
(22) Date of filing: 17.11.2020
(51) Int. Cl.: C12N 9/02, C12N 1/20, C12Q 1/22, A61L 2/00

(54) **BIOLOGICAL INDICATOR FOR RAPID VERIFICATION OF DISINFECTION OR STERILIZATION**

(30) Priority: 23.10.2020 KR 20200137919
(71) Applicant: Aden Hygiene, Ent., Cheongju-si, Chungcheongbuk-do 28160 (KR)
(72) Inventor: CHOI, Woo-Sung, 16507 Gyeonggi-do (KR)
(74) Representative: Schweitzer, Klaus

(57) **Abstract**

The present invention provides a biological indicator for verification of disinfection or sterilization, and in particular, provides a biological indicator capable of verifying disinfection or sterilization using luminescent microorganisms, and a kit for verification of disinfection or sterilization using the biological indicator. According to the present invention, it is possible to provide a biological indicator for verification of disinfection or sterilization that may confirm in real time how much disinfection or sterilization has been performed after disinfecting or sterilizing harmful microorganisms by irradiation with UV rays.

## Description

### [RELATED APPLICATIONS]

This application claims priority to Korean Patent Application No. 10-2020-0137919, filed on October 23, 2020 in the Korean Intellectual Property Office, the entire disclosure of which is incorporated herein by reference.

### [BACKGROUND OF THE INVENTION]

### 1. Field of the Invention

The present invention relates to a biological indicator for verification of disinfection or sterilization, and more specifically, to a biological indicator which may rapidly verifying results of disinfection or sterilization using luminescent microorganisms, and a kit for verification of disinfection or sterilization using the biological indicator.

### 2. Description of the Related Art

In modern society, infectious diseases are becoming a big element that threatens human's health and social safety. In the 2000s, infectious diseases such as SARS (2002), Influenza A virus subtype (2009), MERS (2015) were prevalent, and from the end of 2019 to the present day, COVID-19 is prevalent around the world. The cycle of these infectious outbreaks is being shortened.

In particular, incidents of nosocomial infections such as antibiotic resistant bacteria and super bacteria are tending to increase, and infectious diseases prevalent in multi-use facilities, such as pathogenic E. coli, pneumococcal, norovirus, influenza, and the like are also tending to increase.

In order to reduce the risk of such infectious diseases, an effective and safe disinfection system is necessary. In particular, market demands for an ultraviolet UV-C sterilizer without residual problems of chemical substances are increased so that people can safely use multiple-use facilities, and cases where autonomous traveling robots are applied to enhance the safety of quarantines are increased.

Meanwhile, demands for verification of whether the quarantine has been performed correctly are increased. As an example of a technique that can be utilized to verify quarantine performance, biomarkers can be used.

For verification of whether sterilization is performed using a biological indicator, sterilization results may be verified through physical, chemical or biological indicators by a verification method after performing sterilization. Among them, a verification method using the biological indicator to determine results by measuring a sterilization level of an actual strain is highly reliable.

However, unlike other verification methods that can be determined immediately after performing sterilization, the biological indicator has a disadvantage in that it takes several days during cultivation of the strain, thereby causing a delay in determination of whether the sterilization is successfully performed. Nevertheless, since the verification method using the biological indicator is highly reliable, an adoption rate thereof is overwhelmingly high.

Therefore, in order to enhance the efficiency and success rate of quarantine, a development of a reliable biological indicator having a high reliability while enabling to verify sterilization in real time is urgently required.

### [Prior Art Document]

### [Patent Document]

(Patent Document 1) Korean Patent Registration No. 10-1376795

### [SUMMARY OF THE INVENTION]

In consideration of the above-mentioned circumstances, it is an object of the present invention to provide a biological indicator (bioindicator) which may rapidly verify results of disinfection or sterilization in real time.

In addition, another object of the present invention is to provide a kit for verification of disinfection or sterilization, which includes the biological indicator.

In order to achieve the above-described objects, according to an aspect of the present invention, there is provided a biological indicator for rapid verification of disinfection or sterilization.

In addition, according to another aspect of the present invention, there is provided a kit for verification of disinfection or sterilization, which includes the biological indicator.

The biological indicator for rapid verification of disinfection or sterilization includes a luminescent microorganism or a fragment of the luminescent microorganism.

The luminescent microorganism may be a microorganism recombined by inserting a self-luminous bacteria such as Aliivibrio fischeri (A.fischeri), Photobacterium or Xenorhabdus, or a luminescent gene.

Alternatively, the luminescent microorganism may be obtained by inserting a luminescent gene into a strain capable of forming a spore, and the luminescent gene may be inserted before or after the spore is formed in the strain.

A representative example of the luminescent gene in the present invention may be a gene that encodes a luciferase enzyme or a green fluorescent protein, and preferably is a gene that encodes the luciferase enzyme, but it is not limited thereto.

The principle of luminescence through luciferase is that when luciferin, which is a luminescent material, is activated by ATP, luciferase oxidizes the same to make luciferin oxide, and light is generated in this process.

A recombinant cell refers to a cell that replicates a heterologous nucleic acid, expresses the nucleic acid, or expresses a protein encoded by a peptide, a heterologous peptide, or a heterologous nucleic acid. The recombinant cell may express a gene or a fragment of the gene which is not found in a natural form of the cell in either a sense or antisense form. Further, the recombinant cell may express a gene found in the cell in its natural state, but the gene may be modified and reintroduced into the cell by an artificial means. The recombination method may use transformation, transduction, electroporation, heat shock, and the like, which are widely known in the art.

A vector required for the recombination is used when referring to DNA fragment(s) or nucleic acid molecules to be delivered into the cell. The vector may replicate DNA and may be reproduced independently in a host cell. In this regard, an expression vector is often used interchangeably with the recombinant vector. In addition, the recombinant vector means a recombinant DNA molecule which includes a desired coding sequence, and an appropriate nucleic acid sequence necessary for expressing coding sequences operably linked to each other in a specific host organism. Promoters, enhancers, termination signals and polyadenylation signals which can be used in eukaryotic cells are known in the art.

As used herein, the term "transformation" means that DNA is introduced into a host cell such that the DNA can be replicated as an extrachromosomal factor or by completion of chromosomal integration. A transformant of the present invention may be inserted into the chromosome by causing homologous recombination with a sequence of an endogeneous gene site in the host cell genome by a sequence of the inserted gene, after a vector is transformed into a host strain, or may be held in a form of a plasmid. In addition, the transformation may be performed by a general method used in the art when inserting a gene into the strain.

The recombinant microorganism has a strong resistance to disinfection or sterilization, but has a low resistance to UV, thereby it should be a microorganism that may be sterilized or sterilized with UV.

The UV may be a UV-A, UV-B, or UV-C, but UV-C having a wavelength of 100 to 280 nm is preferably used.

The microorganism may be an intestinal microorganism, a respiratory microorganism, or an oral microorganism, but it is not limited thereto.

The intestinal microorganism may be, for example, a microorganism belonging to Bacteroidetes, Firmicutes, Actinobacteria, Proteobacteria, Verrucomicrobia, or Fusobacteria phylum. The microorganism belonging to the Bacteroidetes phylum may be a microorganism belonging to the Bacteroidaceae, Prevotellaceae, Rikenellaceae, or Porphyromonadaceae family.

The microorganism belonging to the Bacteroidetes phylum may be a microorganism belonging to Bacteroides, Xylanibacter, Alistipes, or Parabacteroides genus. The microorganism belonging to the Bacteroidetes phylum may be may be a microorganism belonging to B.fragilis, B. melaninogenicus, B. oralis, B. vulgatus, B. acidifaciens, B. thetaiotaomicron, B. caccae, B. stercoris, B. coprocola, B. eggerthii, B. intestinalis, B. massiliensis, B. ovatus, B. plebeius, B. uniformis, B. galacturonicus, B. merdae, B. capillosus, P. intermedia, P. aurantiaca, P. copri, P.nigrescens, P. brevis, P. denticola, P. heparinolytica, P. paludivivens, P. ruminicola, X. oryzae, A.finegoldii, A. indistinctus, A. putredinis, A. onderdonkii, but it is not limited thereto.

The microorganism belonging to the Firmicutes phylum may be a microorganism belonging to Lactobacillaceae, Ruminococcaceae, Lachnospiraceae, Leuconostocaceae, Peptostreptococcaceae, Veillonellaceae, Clostridiaceae, Enterococcaceae, Streptococcaceae, Staphylococcaceae, Eubacteriaceae, Bacillaceae, or Butyrate-producing bacterium family.

The microorganism belonging to the Firmicutes phylum may be a microorganism belonging to Pediococcus, Lactobacillus, Ruminococcus, Subdoligranulum, Roseburia, Acetitomaculum, Coprococcus, Leuconostoc, Weissella, Peptostreptococcus, Veillonella, Faecalibacterium, Clostridium, Dorea, Anaerotruncus, Enterococcus, Streptococcus, Lactococcus, staphylococcus, Anaerofustis, Eubacterium, Bacillus.

The microorganism belonging to the Firmicutes phylum may be a microorganism belonging to P. acidolactis, P. pentosaceus, L. plantarum, L. acidophilus, L.Paracasei, L. reuteri, L. helveticus, L. rhamnosus, L. Bulgaricus, L. casei, L. fermentum, L. gasseri, L. helveticus, L. johnsonii, L. salivarius, R. obeum, R. gnavus, R. torques, R. bromii, R. lactaris, Subdoligranulum sp. DJF VR33k2, S. variabile, R. intestinalis, R. hominis, A. ruminis, C. eutactus, C. comes, L. mesenteroides, L. carnosum, L. citreum, L. gasicomitatum, L. gellidum, L. inhae, L.kimchii, L. lactis, L. mesenteroides subsp.mesenteroides, L. paramesenteroides, W. cibaria, W.confusa, W. koreensis, W. soli, W. viridescens, P. anaerobius, V. parvula, F. prausnitzii, C.perfringens, C. difficile, C. botulinum, C. tetani, C. septicum, C. asparagiforme, C. butyricum, C.bolteae, C. leptum, C. orbiscindens, C. saccharolyticum, C. scindens, C. asparagiforme, C. nexile, D.longicatena, D. formicigenerans, A. colihominis, E. faecalis, E. faecium, S. thermophilus, S.pyogenes, S. faecium, S. faecalis, L. lactis, S. aureus, A. stercorihominis, E. tenue, E.ventriosum, E. rectale, E. eligens, E. siraeum, E. hallii, B. cereus, B. subtilis, B. coagulans, B.mesentericus, B. licheniformis, B. polyfermenticus, Butyrate-producing bacterium A1-86, Butyrateproducing bacterium A2-207, Butyrate-producing bacterium M21/2, Butyrate-producing bacterium SL6/1/1, Butyrate-producing bacterium SSC/2, but it is not limited thereto.

The microorganism belonging to the Actinobacteria phylum may be a microorganism belonging to Bifidobacteriaceae, Mycobacteriaceae, or Propionibacteriaceae family.

The microorganism belonging to the Actinobacteria phylum may be a microorganism belonging to Bifidobacterium, Mycobacterium, or Propionibacterium genus.

The microorganisms belonging to the Actinobacteria phylum may be a microorganism belonging to B. breve, B. bifidum, B. longum, B. infantis, B.pseudolongum, B. lactis, B. animalis, B. adolescentis, B. pseudocatenulatum, B. pullorum, B.ruminantium, B. simiae, B. thermophilum, M. avium spp.paratuberculosis, but it is not limited thereto.

The microorganism belonging to the Proteobacteria phylum may be a microorganism belonging to the Enterobacteriaceae, Helicobacteraceae, Desulfovibrionaceae, Alcaligenaceae, Vibrionaceae, Pseudomonadaceae, or Campylobacteraceae family.

The microorganism belonging to the Proteobacteria phylum may be a microorganism belonging to Salmonella, Klebsiella, Enterobacter, Shigella, Escherichia, Yersinia, Proteus, Edwardsiella, helicobacter, Desulfovibrio, Bordetella, Vibrio, Pseudomonas, or Campylobacter genus genus.

The microorganism belonging to the Proteobacteria phylum may be a microorganism belonging to S. enterica subsp. Entericaserovar, S. typhimurium, S. enteritidis, K. pneumoniae, K. oxytoca, E. cloacae, E. aerogenes, S.flexneri, S. dysenteriae, S. boydii, S. sonni, E. coli, E.coli O157, Y. enterocolitica, Y. pestis, Y. pseudotuberculosis, P. mirabilis, E. tarda, H. hepaticus, H. pylori, D. desulfuricans, B.pertussis, V. cholerae, V. parahemolyticus, V. vulnificus, P. aeruginosa, but it is not limited thereto.

The microorganism belonging to the Verrucomicrobia phylum may be a microorganism belonging to Verrucomicrobiaceae family. The microorganism belonging to the Verrucomicrobia phylum may be a microorganism belonging to Akkermansia genus.

The microorganism belonging to the Verrucomicrobia phylum may be a microorganism belonging to the A.muciniphila species, but it is not limited thereto. The microorganism belonging to the Fusobacteria phylum may be a microorganism belonging to Fusobacteriaceae family.

The microorganism belonging to the Fusobacteria phylum may be a microorganism belonging to Fusobacterium genus.

The microorganism belonging to the Fusobacteria phylum may be a microorganism belonging to F. nucleatum, F. mortiferum, or F. necrophorum species, but it is not limited thereto.

A representative example of the oral microorganism may include Porphyromonas gingivalis, Tannerella forsythia, and Treponema denticola, but it is not limited thereto.

In one embodiment of the present invention, the intestinal microorganism may be Salmonella enterica, the respiratory microorganism may be Acinetobacter baumannii, and the oral microorganism may be Porphyromonas gingivalis, but it is not limited thereto.

In the present invention, in order to apply to a sterilization system, the kit for verification of sterilization including the biological indicator as described above may be commercialized and manufactured by a configuration including a thin film type UV-ray transmittance cover, a spore carrier, and a floor packaging material.

A material of the UV-ray transmittance cover should be a material that does not deteriorate the transmittance of UV rays, and quartz is preferably used.

The spore carrier may be inoculated with 10,000 cfu/ml to 1,000,000 cfu/ml of the luminescent microorganism, a spore type luminescent microorganism or fragments thereof according to the present invention. The inoculated luminescent microorganism may be included in a dried form, but it is not limited thereto.

A material of the spore carrier is not limited, but stainless steel, cellulose paper, polystyrene or polypropylene is preferably used.

In addition, the spore carrier is divided into an inoculation surface and a non-inoculation surface, and the inoculation surface may come into contact with the UV-ray transmittance cover.

A material of the floor packaging material may use a material commonly used in manufacturing the floor packaging material in the related art, and it is not limited to a specific material.

The present invention may provide a biological indicator for verification of disinfection or sterilization, and in particular, provide a biological indicator capable of verifying disinfection or sterilization using luminescent microorganisms, and a kit for verification of disinfection or sterilization using the biological indicator. According to the present invention, it is possible to provide a biological indicator for verification of disinfection or sterilization that may confirm in real time how much disinfection or sterilization has been performed after disinfecting or sterilizing harmful microorganisms by irradiation with UV rays.

### [BRIEF DESCRIPTION OF THE DRAWINGS]

The above and other objects, features and other advantages of the present invention will be more clearly understood from the following detailed description taken in conjunction with the accompanying drawings, in which:

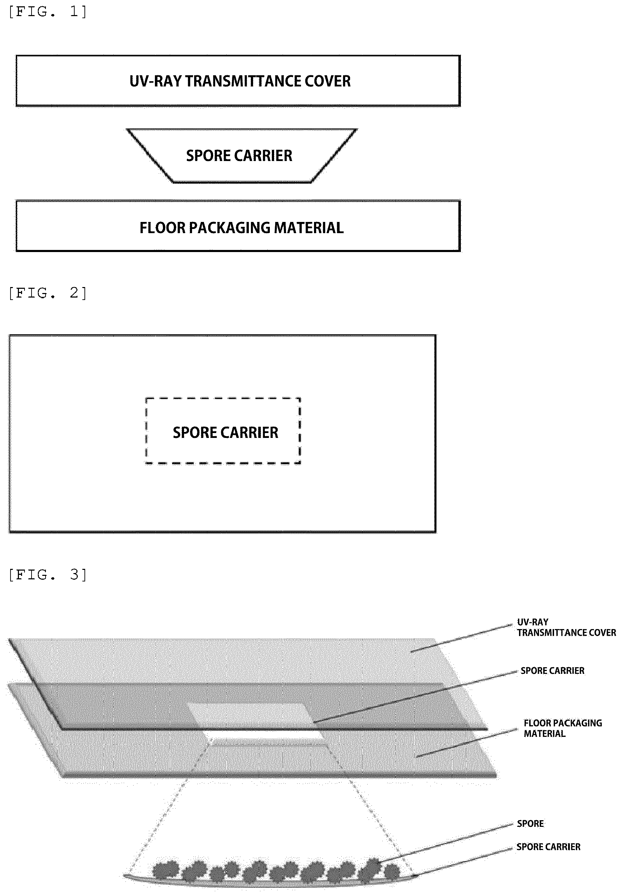

FIG. 1 is an enlarged view illustrating a configuration of a kit for verification of sterilization by irradiation with UV rays, which includes a biological indicator according to the present invention;
FIG. 2 is a plan view of the configuration shown in FIG. 1; and
FIG. 3 is a three-dimensional view of the configuration shown in FIG. 1.

### [DETAILED DESCRIPTION OF THE INVENTION]

Hereinafter, the present invention will be described in detail by examples and experimental examples.

However, the following examples and experimental examples are only given for illustrating the present invention, and contents of the present invention are not limited to the following examples and experimental examples.

### <Example 1> Preparation of biological indicator

Intestinal microorganism Salmonella enterica was selected as a test strain, and Acinetobacter baumannii was selected as a respiratory microorganism, then a biological indicator was prepared by the following manner.

First, luciferase gene (pMCS-Red Firefly Luc Vector for Luciferase Assays (16155), ThermoFisher; pMCS-Gaussia Luc Vector for Luciferase Assays (16146), ThermoFisher) was subjected to ligation, followed by applying heat shock for transformation. After sequencing the vector, an activated cell (competent cell) was prepared so that DNA could be inserted. Thereafter, the cell was transformed using electroporation, followed by confirming whether the cell is transformed through colony-PCR.

### <Example 2> Preparation of biological indicator

The intestinal microorganism Salmonella enterica was selected as a test strain, and a biological indicator was prepared in the same manner as in Example 1.

### <Example 3> Preparation of a biological indicator

A biological indicator was prepared according to the same procedures as described in Example 1, except that oral microorganism Porphyromonas gingivalis was selected as a test strain.

<Experimental Example 1> Confirmation of sterilization verification effect by measuring an amount of bioluminescence expression of the biological indicator according to the present invention

An in-vitro experiment was performed to confirm a sterilization verification effect of the biological indicator including the intestinal microorganism and the respiratory microorganism prepared in Example 1. First, E. coli, and the like were smeared on a medium, and whether there is luminescence was measured before and after sterilization with UV-C having a wavelength of 100 to 280 nm. A luminescence measurement was performed through a primary observation using a simple inspection equipment utilizing a UV lamp (420 to 680 nm) in the field, and when clear quantitative evaluation was not possible in the primary observation, the luminescence was quantitatively measured using a charge-cooled digital (CCD) camera equipment. As a result of the experiment, it was immediately confirmed that, before sterilization with UV-C, luminescence of the biological indicator prepared in Example 1 was exhibited in a wide range, but after sterilization with UV-C, the luminescence range of the biological indicator was decreased.

<Experimental Example 2> Confirmation of sterilization verification effect by measuring an amount of bioluminescence expression of the biological indicator according to the present invention

An in-vivo experiment was performed to confirm a sterilization verification effect of the biological indicator including the intestinal microorganism prepared in Example 2. A mouse was prepared, and the biological indicator was administered into an abdomen of the mouse, then whether there is luminescence was measured measured before and after sterilization with UV-C having a wavelength of 100 to 280 nm. A luminescence measurement was performed through a primary observation using a simple inspection equipment utilizing a UV lamp (420 to 680 nm) in the field, and when clear quantitative evaluation was not possible in the primary observation, the luminescence was quantitatively measured using a charge-cooled digital (CCD) camera equipment. As a result of the experiment, it was immediately confirmed that, before sterilization with UV-C, luminescence of the biological indicator prepared in Example 2 was exhibited in a wide range, but after sterilization with UV-C, the luminescence range of the biological indicator was decreased.

<Experimental Example 3> Confirmation of sterilization verification effect by measuring an amount of bioluminescence expression of the biological indicator according to the present invention

An in-vitro experiment was performed to confirm a sterilization verification effect of the biological indicator including various types of oral microorganisms prepared in Example 3. First, a sample containing E. coli and the like and the biological indicator prepared in Example 3 were put together in a tube and sterilized with UV-C having a wavelength of 100 to 280 nm, and whether there is luminescence was measured before and after sterilization with UV-C having a wavelength of 100 to 280 nm. A luminescence measurement was performed through a primary observation using a simple inspection equipment utilizing a UV lamp (420 to 680 nm) in the field, and when clear quantitative evaluation was not possible in the primary observation, the luminescence was quantitatively measured using a charge-cooled digital (CCD) camera equipment. As a result of the experiment, it was immediately confirmed that, before sterilization with UV-C, luminescence of the biological indicator prepared in Example 3 was exhibited in a wide range, but after sterilization with UV-C, the luminescence range of the biological indicator was decreased.

The kit for verification of disinfection or sterilization, which includes the biological indicator according to the present invention may provide a reliable biological indicator while being capable of performing verification in real time. Further, the biological indicator and the kit may be used to accurately check quarantine results in real time, such that industrial applicability is very high.

## Claims

1. A biological indicator for rapid verification of disinfection or sterilization comprising a luminescent microorganism or a fragment of the luminescent microorganism.

2. The biological indicator for rapid verification of disinfection or sterilization according to claim 1, wherein the biological indicator verifies sterilization by irradiation with UV rays.

3. The biological indicator for rapid verification of disinfection or sterilization according to claim 1, wherein the luminescent microorganism is transformed by inserting a luciferase gene into a microorganism.

4. The biological indicator for rapid verification of disinfection or sterilization according to claim 3, wherein the luciferase gene is inserted before or after spore formation of the microorganism.

5. The biological indicator for rapid verification of disinfection or sterilization according to claim 3, wherein the luminescent microorganism is transformed by inserting a luciferase gene into any one or more strains selected from Salmonella enterica, Acinetobacter baumannii, and Porphyromonas gingivalis.

6. A kit for verification of disinfection or sterilization comprising a UV-ray transmittance cover and a spore carrier.

7. The kit for verification of disinfection or sterilization according to claim 6, wherein the spore carrier is inoculated with 10,000 cfu/ml to 1,000,000 cfu/ml of a luminescent microorganism, a spore type luminescent microorganism, or fragments thereof.

8. The kit for verification of disinfection or sterilization according to claim 6, wherein a material of the spore carrier is any one of stainless steel, cellulose paper, polystyrene, or polypropylene.

9. The kit for verification of disinfection or sterilization according to claim 6, wherein a material of the UV-ray transmittance cover is quartz.
